# EUROPÄISCHE PATENTSCHRIFT

(11) **EP 3 821 840 B1**
(45) Veröffentlichungstag und Bekanntmachung des Hinweises auf die Patenterteilung: **18.03.2026**
(21) Anmeldenummer: 19208558.7
(22) Anmeldetag: 12.11.2019
(51) Int. Cl.: A61B 18/18, A61N 1/40, A61N 5/02, A61N 5/04

(54) **APPLIKATOR ZUM APPLIZIEREN EINES ELEKTROMAGNETISCHEN FELDES IN KÖRPERGEWEBE**
APPLICATOR FOR APPLYING ELECTROMAGNETIC FIELD TO TISSUE
APPLICATEUR POUR APPLIQUER UN CHAMP ÉLECTROMAGNÉTIQUE AU TISSU

(43) Veröffentlichungstag der Anmeldung: 19.05.2021
(73) Patentinhaber: Gbo Medizintechnik AG, 64668 Rimbach (DE)
(72) Erfinder: KECK, Eberhard, 69509 Mörlenbach (DE); VEIT, Thomas, 69483 Wald-Michelbach (DE)
(74) Vertreter: Franke, Markus

(56) Entgegenhaltungen:
- EP-A2- 2 780 080
- KR-B1- 101 710 313
- US-A- 4 926 881
- US-A- 6 047 215
- US-A1- 2014 249 609

## Beschreibung

Die vorliegende Erfindung betrifft einen Applikator zum Applizieren eines elektromagnetischen Feldes in Körpergewebe, insbesondere in subkutanes Fettgewebe. Bei dem applizierten elektromagnetischen Feld handelt es sich insbesondere um Hochfrequenz-Wellen (HF-Wellen). Der erfindungsgemäße Applikator findet vorzugsweise Verwendung bei Hochfrequenztherapie, beispielsweise Kurzwellentherapie oder Mikrowellentherapie. Der Applikator findet insbesondere Anwendung bei einem Gerät zur Fettreduzierung, bspw. durch temperaturinduzierte Zell-Apoptose, wobei die Erwärmung des Fettgewebes durch ein mittels des Applikators appliziertes hochfrequentes elektromagnetisches Feld erreicht wird. Vorzugsweise findet das Gerät bzw. der Applikator auch bei der Wärmetherapie Anwendung. Bei dem mittels des Applikators applizierbaren elektromagnetischen Feldes handelt es sich insbesondere um ein elektromagnetisches Wechselfeld, wobei die Frequenz des Wechselfeldes vorzugsweise 10 MHz bis 50 MHz, insbesondere 13,56 MHz oder 27,12 MHz oder 40,68 MHz, beträgt. Es sind aber auch durchaus Frequenzen im Bereich von 400 MHz bis 500 MHz oder auch 2 GHz bis 3 GHz denkbar.

Aus dem Stand der Technik sind Applikatoren zum Applizieren eines elektromagnetischen Feldes in Körpergewebe, insbesondere in subkutanes Fettgewebe, bekannt, bei denen Elektroden mit dem Körpergewebe in Kontakt gebracht werden. Dabei werden häufig Elektrodengele, auch als Leitgele oder Kontaktgele, bezeichnet, verwendet. Diese weisen eine hohe Leitfähigkeit auf und dienen u.a. zur Verbesserung der Übermittlung elektrischer Impulse entgegen dem Übergangswiderstand der Hautoberfläche ins Körperinnere. Ein solcher Applikator, bei dem die Elektroden bei der Behandlung mit dem Körpergewebe in Kontakt gebracht werden, ist aus der KR 101 710 313 B1 bekannt. Bei diesem ist zur Vereinfachung der Handhabung eine die jeweilige Elektrode umlaufende Dichtlippe vorgesehen, wobei mittels der Dichtlippe ein Vakuum erzeugt werden kann, um die Elektrode mit der Körperoberfläche in Kontakt zu bringen und in Kontakt zu halten. Die US 2014/0249609 A1 offenbart einen Applikator, der die Merkmale des Oberbegriffs des Patentanspruchs 1 aufweist. Die US 4 926 881 A offenbart weiteren Stand der Technik.

Verfahren und Geräte zur Fettreduzierung mittels kontaktloser Applikation von hochfrequenten elektromagnetischen Wellen sind aus dem Stand der Technik bekannt. Beispielsweise beschreibt die EP 2 780 080 A1 ein System zum berührungslosen Behandeln von subkutanem Gewebe eines Patienten, das ein Volumen an lipidreichen Zellen hat. Das System weist einen Applikator mit mindestens einem Paar von zwei symmetrischen kapazitiven Elektroden auf, wobei der Applikator dazu eingerichtet ist, beabstandet von der Haut des zu behandelnden Patienten angeordnet zu sein. Zu diesem Zweck weist der Applikator eine mechanische Halterung auf, die dazu eingerichtet ist, den Applikator temporär in einer Position relativ zu dem Patienten zu fixieren. Der Applikator ist ferner dazu eingerichtet, um die entsprechenden HF-Wellen in das subkutane Gewebe zu übertragen, um dadurch das subkutane Gewebe mittels der HF-Wellen zu erwärmen. Bei dem aus der EP 2 780 080 A1 bekannten Applikator sind die beiden kapazitiven Elektroden jeweils in einem separaten Grundkörper gelagert, wobei die beiden Elektroden bzw. Grundkörper unabhängig voneinander bezüglich des zu behandelnden Patienten positioniert werden müssen. Bei dem Applikator gemäß der EP 2 780 080 A1 handelt es sich somit um einen kontaktlosen Applikator, da der Applikator dazu eingerichtet ist, beabstandet von dem Körper des zu behandelnden Patienten angeordnet zu werden. Die beabstandete Anordnung des Applikators bzw. der in den Grundkörper gelagerten Elektroden von der Körperoberfläche des Patienten hat den Nachteil, dass die Ausrichtung bzw. Justage der Elektroden zueinander und des Abstands der Elektroden von der Körperoberfläche des Patienten mit einem hohen Aufwand verbunden ist, da die zu behandelnden Körperformen zum einen von Patient zu Patient unterschiedlich sind. Zudem unterscheidet sich die Körperform je nach zu behandelnder Region. So ist bei einer Behandlung von Fettgewebe im Bereich des Bauches die Körperform eher gewölbt, wohingegen die Körperform im Bereich des Rückens eher eben ist. Ferner müssen die Elektroden zwecks optimaler Wirkung in einem bestimmten Abstand zu der Körperoberfläche und in einem bestimmten Abstand zueinander angeordnet werden, was zusätzlich den Justageaufwand bzw. Einrichtungsaufwand erhöht. Insofern ist der Zeitaufwand zur Einrichtung bzw. Einstellung des Geräts bzw. Anpassung der Elektrodenanordnung des Geräts auf die jeweils zu behandelnde Körperregion bzw. den jeweils zu behandelnden Patienten sehr zeitaufwendig. Des Weiteren ist nachteilig bei dem Gerät, dass der Patient während der Behandlung seine Position bezüglich der nach Einrichtung ortsfest angeordneten Elektroden gar nicht oder nur in geringem Maße verändern darf, um die angestrebte Wirkung zu erzielen, was für den Patienten unkomfortabel ist.

Aufgabe der vorliegenden Erfindung ist es daher, einen Applikator zum Applizieren eines elektromagnetischen Feldes in Körpergewebe zu schaffen, der die vorgenannten Nachteile überwindet.

Gelöst wird dieses Aufgabe durch einen Applikator, der die Merkmale des Patentanspruchs 1 aufweist.

Der erfindungsgemäße Applikator dient dem Applizieren eines elektromagnetischen Feldes in Körpergewebe, insbesondere in subkutanes Fettgewebe. Der erfindungsgemäße Applikator weist zumindest eine Elektrode und zumindest eine Gegenelektrode auf, wobei der Applikator einen biegsamen Grundkörper aufweist, wobei die zumindest eine Elektrode und die zumindest eine Gegenelektrode in dem Grundkörper gelagert sind. Der Applikator weist auf einer dem Körper zuzuwendenden Seite mehrere Abstandshalter auf, wobei der Applikator Kanäle zum Zuführen und/oder Abführen von Luft aufweist, wobei der Applikator auf der dem Körper zuzuwendenden Seite mehrere mit den Kanälen fluidverbundene Öffnungen aufweist.

Aufgrund der Tatsache, dass die Elektrode und die Gegenelektrode in dem Grundkörper gelagert sind, ist ein Abstand der Elektrode zu der Gegenelektrode festgelegt, sodass eine separate Positionierung der Elektrode bezüglich der Gegenelektrode und umgekehrt entfällt. Aufgrund der gemeinsamen Lagerung von Elektrode und Gegenelektrode in dem Grundkörper werden zudem Positionierungsfehler, beispielsweise ein zu geringer Abstand zwischen Elektrode und Gegenelektrode, vermieden. Somit ist die Handhabung des Applikators vereinfacht und Positionierungsfehler, wie sie beispielsweise bei der Handhabung von zwei separaten Elektroden auftreten können, werden vermieden. Da der die Elektrode und die Gegenelektrode aufnehmende Grundkörper biegsam ist, ist eine Verformung dieser Struktur möglich, wodurch sich der Applikator optimal an die Körperform anpassen lässt. Dabei ist insbesondere vorgesehen, dass der Applikator mit seiner dem Körper zuzuwendenden Seite an der Körperoberfläche, vorzugsweise vollflächig, anliegt, wobei die Abstandshalter die Körperoberfläche kontaktieren. Durch die Abstandshalter ist sichergestellt, dass die in dem Grundkörper gelagerte Elektrode und die in dem Grundkörper gelagerte Gegenelektrode sich in einem definierten Abstand von der Körperoberfläche befinden. Dadurch dass der Applikator an der Körperoberfläche anliegt, kann sich der Patient bewegen bzw. die zu behandelnde Körperregion kann bewegt werden, ohne dass sich diese Bewegung negativ auf die Applikation des Feldes auswirkt, da der Applikator sich der veränderten Körperform anpasst bzw. sich mit dem Körper mitbewegen kann.

Da der Applikator Kanäle zum Zuführen und/oder Abführen von Luft aufweist, welche mit auf der dem zu behandelnden Körper zuzuwendenden Seite ausgebildeten Öffnungen fluidverbunden sind, kann aktiv ein Luftstrom in dem von dem Applikator abgedeckten Bereich der Körperoberfläche erzeugt werden. Die aktive Erzeugung eines Luftstroms hat mehrere Vorteile. Zum einen kann die Körperoberfläche gekühlt werden, wodurch unangenehme Hauttemperaturen, sogenannte Hotspots, vermieden werden. Dadurch wird zum einen die Behandlung für die zu behandelnde Person angenehmer und zum anderen sind auch längere Behandlungszeiten möglich. Ferner ist die Erzeugung eines aktiven Luftstroms in dem Zwischenbereich zwischen Applikator und Körperoberfläche dahingehend vorteilhaft, dass Schweißbildung vermieden oder reduziert wird, ferner die Körperoberfläche durch den Luftstrom getrocknet werden kann, insofern entstandener Schweiß getrocknet wird. Schweißbildung stellt bei der Behandlung mit hochfrequenten elektromagnetischen Wellen ein Problem dar, da sich diese salzhaltige Flüssigkeit stark in dem applizierten Feld erwärmen würde, was zu Verbrennungen führen kann. Ein aktiver Luftstrom, insbesondere ein aktiver Luftstrom mit Luft, die eine Temperatur unterhalb der Körperoberflächentemperatur aufweist, vorzugsweise ein aktiver Luftstrom mit gekühlter Luft, somit Luft, die eine Temperatur unterhalb der Umgebungstemperatur aufweist, hat den Vorteil, dass von außen quasi eine Kühlung der behandelten Körperregion erfolgt, sodass durch intensive Kühlung der Körperoberfläche tieferliegende Gewebeschichten intensiver behandelt werden können, ohne darüberliegende Schichten zu stark zu erwärmen. Die Möglichkeit der aktiven Erzeugung eines Luftstroms ist insbesondere bei einer Anlage des Applikators an der Körperoberfläche, wie sie der erfindungsgemäße Applikator ermöglicht, von Vorteil, da es aufgrund der Abdeckung der Körperoberfläche zu einer unerwünscht starken Erwärmung des äußeren Körpergewebes kommen kann. Ferner ermöglicht eine selektive aktive Kühlung eine selektive Erwärmung von bestimmten Körpergewerberegionen, wodurch die Behandlungsintensität örtlich variiert werden kann. Durch eine entsprechende Anordnung der Öffnung bzw. eine selektive Verteilung der Luftkühlung lässt sich somit quasi ein Body Contouring erzielen. Bspw. ermöglicht es der Applikator durch selektive Kühlung bestimmter Regionen die mit der Behandlung gewünschte Fettreduzierung örtlich zu variieren, um auf diese Weise die Körperregion zu formen.

Vorzugsweise weist der Applikator eine Vielzahl von Öffnungen, zumindest zehn Öffnungen, vorzugsweise zumindest zwanzig Öffnungen auf.

Es wird als besonders vorteilhaft angesehen, wenn der Grundkörper auf der dem Körper zuzuwendenden Seite des Grundkörpers mehr als vierhundert Öffnungen pro Quadratmeter aufweist. Insbesondere weist der Grundkörper zwischen vierhundert und tausend Öffnungen pro Quadratmeter auf, insbesondere neunhundert Öffnungen pro Quadratmeter auf.

Da der erfindungsgemäße Applikator eine aktive Kühlung der durch den Applikator abgedeckten Körperoberfläche ermöglicht, kann der Applikator großflächig ausgebildet sein. Es wird als vorteilhaft angesehen, wenn der Applikator auf seiner dem Körper zuzuwendenden Seite eine Fläche von mindestens 500 cm² aufweist. Vorzugsweise weist der Applikator auf der dem Körper zuzuwendenden Seite eine Fläche von 700 cm² bis 900 cm² auf.

Es wird als besonders vorteilhaft angesehen, wenn die Abstandshalter und/oder die Öffnungen über die gesamte dem Körper zuzuwendende Fläche des Applikators, vorzugsweise regelmäßig, verteilt sind.

Als besonders vorteilhaft wird es angesehen, wenn der Applikator länglich ausgebildet ist. Insbesondere ist vorgesehen, dass der Applikator eine Längserstreckung von 50 bis 100 cm und eine Quererstreckung von 10 bis 40 cm aufweist.

Als besonders vorteilhaft hinsichtlich eines optimalen Luftstroms hat es sich erwiesen, wenn die Kanäle eine Querschnittsfläche von mindestens 0,2 cm², insbesondere eine Querschnittsfläche von 0,25cm² aufweisen. Als besonders vorteilhaft hinsichtlich eines optimalen Luftstroms hat es sich erwiesen, wenn die Kanäle eine Querschnittsfläche von höchstens 1 cm² aufweisen.

Zwecks Zuführen und Abführen von Luft mittels der Öffnungen und Kanäle wird es als vorteilhaft angesehen, wenn der Applikator zumindest einen Anschluss aufweist, wobei der Anschluss mit den Kanälen fluidverbunden ist, wobei der Anschluss mit einer Einrichtung zum Zuführen und/oder Abführen von Luft verbindbar ist. Vorzugsweise ist dieser Anschluss auf einer dem Körper abzuwendenden Seite des Applikators angeordnet.

Um die Kühlwirkung des Luftstroms und/oder ein Trocknen der Körperoberfläche zu verbessern, wird es als vorteilhaft angesehen, wenn in der jeweiligen Öffnung eine Luftleiteinrichtung zum Umlenken und/oder Aufteilen des Luftstroms angeordnet ist.

Vorzugsweise ist die Luftleiteinrichtung derart ausgebildet, dass der umgelenkte Luftstrom, bezogen auf die Körperoberfläche, eine Tangentialkomponente und eine Normalkomponente aufweist, wobei der Betrag der Tangentialkomponente größer ist als der Betrag der Normalkomponente. Vorzugsweise wird die Körperoberfläche im Wesentlichen tangential angeströmt.

Als besonders vorteilhaft wird es angesehen, wenn die Luft zwischen die Abstandshalter geleitet wird.

Bei der Elektrode und der Gegenelektrode handelt es sich vorzugsweise um Kondensatorelektroden.

Hinsichtlich der Luftleiteinrichtung wird es als besonders vorteilhaft angesehen, wenn die Luftleiteinrichtung als separates Teil ausgebildet ist und vorzugsweise lösbar in der Öffnung gelagert ist. Dabei wird es als vorteilhaft angesehen, wenn die Luftleiteinrichtung in den Grundkörper eingesteckt oder eingeknöpft ist. Die Ausbildung der Luftleiteinrichtung als separates Teil hat den Vorteil, dass in einfacher Art und Weise ein Austausch der Luftleiteinrichtung gegen eine anders gestaltete Luftleiteinrichtung möglich ist, bei sonst unverändertem Applikator. Dadurch ist es möglich, den Applikator an die gewünschte Kühlwirkung optimal anzupassen. In diesem Zusammenhang sei darauf hingewiesen, dass es durchaus denkbar ist, bestimmte Öffnungen mittels Stopfen zu verschließen, sodass die der verschlossenen Öffnung benachbarte Körperoberfläche weniger stark gekühlt wird als andere Körperoberflächen.

Die Abstandshalter sind derart ausgebildet, dass bei Anlage des Applikators an dem Körper ein Strömen von Luft von außerhalb in den Bereich zwischen Applikator und Körperoberfläche und/oder ein Strömen von Luft zwischen Applikator und Körperoberfläche nach außerhalb möglich ist, vorzugsweise ein Strömen von Umgebungsluft in den Bereich zwischen Applikator und Körperoberfläche und/oder umgekehrt möglich ist. Diese Gestaltung der Abstandshalter ermöglicht somit ein Ansaugen von Umgebungsluft in den Bereich zwischen Applikator und Körperoberfläche bzw. eine Ausströmen von Luft aus dem Bereich zwischen Applikator und Körperoberfläche.

Die Abstandshalter sind vorzugsweise als Noppen ausgebildet.

Hinsichtlich der Verwendung des Applikators hat es sich als besonders vorteilhaft erwiesen, wenn der Applikator derart verwendet wird, dass aktiv Luft durch die Kanäle und die mit den Kanälen fluidverbundenen Öffnungen in Richtung der Körperoberfläche geblasen wird.

In einer besonders bevorzugten Ausführungsform des Applikators ist vorgesehen, dass der Applikator eine Erstreckung in Längsrichtung und eine Erstreckung in Querrichtung aufweist, wobei die Erstreckung in Längsrichtung größer ist als die Erstreckung in Querrichtung, wobei die Elektrode in der Querrichtung beabstandet von der Gegenelektrode angeordnet ist.

Vorzugsweise erstrecken sich die Elektrode und die Gegenelektrode im Wesentlichen in Längsrichtung des Applikators. Ein wesentlicher Vorteil der Ausrichtung der Elektroden in der vorgenannten Art ist, dass dadurch eine Anordnung der Elektrode und Gegenelektrode senkrecht zur Körperachse (Elektrode oben, Gegenelektrode unten oder umgekehrt) in möglich ist, wobei bei einer derartigen Anordnung Randbereiche des Körpers wenig erwärmt werden. Speziell bei der Bauchanlage ist so sichergestellt, dass die eher empfindlichen Knochen mit Knochenmark (Rippenbogen, Hüftknochen) nicht unnötig erwärmt werden.

Der Abstand zwischen der Elektrode und der Gegenelektrode beträgt insbesondere 30 mm bis 100 mm, vorzugsweise zumindest 40 mm.

Als besonders vorteilhaft wird es angesehen, wenn die Elektrode und die Gegenelektrode spiegelsymmetrisch angeordnet sind. Vorzugsweise sind die Elektrode und die Gegenelektrode spiegelsymmetrisch zu einer Ebene angeordnet, die sich in Längsrichtung des Applikators und senkrecht zur Querrichtung des Applikators erstreckt.

Die Elektrode und die Gegenelektrode sind vorzugsweise aus Kupfer oder einer Kupfer-Zinn-Legierung gefertigt. Es ist aber auch durchaus denkbar, die Elektroden aus einem anderen, vorzugsweise hoch leitfähigen Material zu fertigen, bspw. einem Edelmetall.

In einer bevorzugten Ausführungsform ist vorgesehen, dass die Elektrode und die Gegenelektrode biegsam sind. Dadurch, dass die Elektrode, die Gegenelektrode und der die Elektrode und die Gegenelektrode aufnehmende Grundkörper biegsam sind, ist eine Verformung dieser Strukturen möglich, wodurch sich der Applikator optimal an die Körperform anpassen lässt.

Als besonders vorteilhaft wird es angesehen, wenn die Elektrode und die Gegenelektrode als biegsame Metallstreifen oder Metallplatten ausgebildet sind. Es ist auch durchaus denkbar, dass die Elektrode und die Gegenelektrode jeweils aus mehreren Metallstreifen gebildet sind, die elektrisch miteinander verbunden sind. Die einzelnen Metallstreifen als solche können durchaus starr sein, wobei durch deren Anordnung und/oder Lagerung und/oder Verbindung die Biegsamkeit der Elektrode bzw. Gegenelektrode erreicht wird.

Vorzugsweise handelt es sich bei der Elektrode und/oder der Gegenelektrode um eine Netzelektrode oder Folienelektrode. Besonders bevorzugt sind die Elektrode und die Gegenelektrode als Metallgaze ausgebildet. Dadurch sind die Elektrode und die Gegenelektrode besonders biegsam und es wird zudem ein vorteilhafte Feldstärkeverteilung erreicht.

Die Elektrode und/oder die Gegenelektrode weisen in einer bevorzugten Ausführungsform eine kammartige Gestaltung auf.

Die Dicke der Gegenelektrode und der Elektrode liegt vorzugsweise zwischen 0,1 mm und 5 mm, besonders bevorzugt zwischen 0,2 mm und 2,0 mm, insbesondere zwischen 0,3 mm und 1,0 mm. Vorzugsweise haben Drähte einer als Metallgaze ausgebildeten Elektrode bzw. Gegenelektrode eine Dicke von 0,1 mm bis 0,2 mm, vorzugweise eine Dicke von 0,125 mm. Eine Maschenweite einer als Metallgaze oder Netzelektrode ausgebildeten Elektrode bzw. Gegenelektrode beträgt vorzugsweise zwischen 0,1 mm und 0,3 mm, insbesondere 0,18 mm.

Vorzugsweise weist der Applikator einen zwischen zwei Endabschnitten ausgebildeten zentralen Abschnitt auf, wobei die Elektrode und die Gegenelektrode derart ausgebildet sind, dass, bei Verwendung des Applikators, im Bereich des zentralen Abschnitts des Applikators eine stärkere Erwärmung des Körpergewebes erreicht werden kann als im Bereich der beiden Endabschnitte. Dadurch kann eine intensivere Erwärmung des Körpergewebes in dem Bereich erreicht werden, der von dem zentralen Abschnitt überdeckt wird. Zu diesem Zweck können die Elektrode und die Gegenelektrode im zentralen Bereich eine größere Flächenausdehnung aufweisen.

Vorzugsweise sind die Elektrode und/oder die Gegenelektrode einteilig ausgebildet.

Vorzugsweise weist der Applikator genau eine Elektrode und genau eine Gegenelektrode auf.

Um die Handhabung des Applikators zu vereinfachen, wird es als vorteilhaft angesehen, wenn der Applikator einen ersten elektrischen Anschluss für die Elektrode, einen zweiten elektrischen Anschluss für die Gegenelektrode und zumindest einen Anschluss zum Zuführen und/oder Abführen von Luft in die Kanäle und/oder aus den Kanälen aufweist, wobei sämtliche Anschlüsse in einer gemeinsamen Lagerstruktur gelagert sind. Dadurch wird das Verbinden des Applikators mit den entsprechenden Leitungen des HF-Geräts vereinfacht.

Als besonders vorteilhaft wird es angesehen, wenn die gemeinsame Lagerstruktur in einem zentralen Bereich des Applikators ausgebildet ist. Dadurch sind die entsprechenden Anschlüsse besonders einfach zugänglich und zudem erleichtert eine derartige Gestaltung die Positionierung und Ausrichtung des Applikators an dem Körper, da die zentrale Lagerstruktur ein optisches Hilfsmittel hinsichtlich der Positionierung darstellt. Wenn beispielsweise Gewebe im Bereich des Torsos behandelt werden soll, beispielsweise Bauchfettgewebe, ist die zentrale Lagerstruktur typischerweise im Bereich des Bauchnabels anzuordnen.

Die Lagerstruktur ist vorzugsweise als Steckbuchse oder Stecker ausgebildet. Somit ist ein einfaches Verbinden der Anschlüsse möglich, da lediglich ein zu der Steckbuchse korrespondierender Stecker mit der Steckbuchse verbunden werden muss bzw. eine zu dem Stecker korrespondierende Steckbuchse mit dem Stecker verbunden werden muss.

Vorzugsweise ist die Lagerstruktur auf einer dem Körper abzuwendenden Seite ausgebildet. Die Lagerstruktur besteht vorzugsweise aus Polypropylen, um eine ausreichende Steifheit der Lagerstruktur zu gewährleisten.

Die Lagerstruktur ist vorzugsweise als Buchse ausgebildet, wobei ein Stecker lösbar mit der Buchse verbindbar ist, wobei der Stecker mit einer ersten elektrischen Leitung für die Elektrode, einer zweiten elektrischen Leitung für die Gegenelektrode und einer Leitung für den zumindest einen Anschluss zum Zuführen und/oder Abführen von Luft verbunden ist, wobei sämtliche Leitungen in einer schlauchartigen, biegsamen Führungsstruktur gelagert sind. Alternativ und bevorzugt ist die Lagerstruktur als Stecker ausgebildet, wobei eine Buchse lösbar mit dem Stecker verbindbar ist, wobei die Buchse mit einer ersten elektrischen Leitung für die Elektrode, einer zweiten elektrischen Leitung für die Gegenelektrode und einer Leitung für den zumindest einen Anschluss zum Zuführen und/oder Abführen von Luft verbunden ist, wobei sämtliche Leitungen in einer schlauchartigen, biegsamen Führungsstruktur gelagert sind. Die Ausführungsform mit einer führungsstrukturseitigen Buchse hat den Vorteil, dass bei einer Handhabung der führungsstrukturseitigen Buchse ein unbeabsichtigter Kontakt mit Kontaktelementen der Buchse, die mit den Leitungen verbunden sind, vermieden wird, wodurch die Buchse besonders sicher gehandhabt werden kann, insbesondere bei mit Leistung beaufschlagten Kontaktelementen. Dabei wird es als vorteilhaft angesehen, wenn die Kontaktelement gegenüber einer Fronseite der Buchse zurückgesetzt ausgebildet sind.

Die Führungsstruktur weist vorzugsweise einen ersten Kanal für die erste elektrische Leitung und einen zweiten Kanal für die zweite elektrische Leitung auf. Durch die Verwendung von Kanälen für die erste elektrische Leitung und die zweite elektrische Leitung sind die Leitungen in der Führungsstruktur definiert gelagert, wodurch die Handhabung erleichtert ist. Bei elektrischen Leitungen, die unabhängig voneinander beweglich sind, muss die Bedienperson stets darauf achten, dass sich die Leitungen nicht kreuzen, einen ausreichenden Abstand halten und nicht in die Nähe von metallischen Gegenständen gelangen. Da die Leitungen definiert in der Führungsstruktur geführt sind, werden Fehler bei der Leitungsführung vermieden. Die Führungsstruktur weist vorzugsweise eine Abschirmung zum Abschirmen des elektrischen Feldes auf.

Es ist durchaus denkbar, dass die Führungsstruktur in einem Gestell geführt ist, um die Handhabung zu vereinfachen.

Als besonders vorteilhaft hinsichtlich einer Fertigung des Applikators wird es angesehen, wenn der Grundkörper aus mehreren Lagen gebildet ist, wobei eine erste Lage des Grundkörpers auf einer ersten Seite mehrere Vorsprünge aufweist, wobei die Vorsprünge die Abstandshalter bilden, und die erste Lage auf einer der ersten Seite abgewandten Seite mehrere Rücksprünge aufweist, wobei die Rücksprünge Teilbereiche der Kanäle bilden.

Vorzugsweise weist der Grundkörper eine zweite Lage auf, wobei die Elektrode und die Gegenelektrode zwischen der ersten Lage und der zweiten Lage angeordnet sind. Als besonders vorteilhaft wird es angesehen, wenn die Elektrode und die Gegenelektrode ohne separate Hilfsmittel zwischen der ersten Lage und der zweiten Lage gehalten sind.

In diesem Zusammenhang wird es als besonders vorteilhaft angesehen, wenn die zweite Lage Aussparungen aufweist, wobei die Elektrode und die Gegenelektrode in den Aussparungen angeordnet sind.

Es ist auch denkbar und bevorzugt, dass der Grundkörper eine Zwischenlage aufweist, wobei die Zwischenlage zwischen der ersten und der zweiten Lage angeordnet ist und die Elektrode und die Gegenelektrode zwischen der Zwischenlage und der zweiten Lage angeordnet sind. Bei der Verwendung einer Zwischenlage ist es durchaus denkbar, dass die Zwischenlage Aussparungen aufweist, wobei die Elektrode und die Gegenelektrode in den Aussparungen angeordnet sind.

Vorzugsweise ist die Zwischenlage derart ausgebildet, dass die Zwischenlage die Rücksprünge abdeckt.

Es ist durchaus denkbar, dass die erste Lage einen umlaufenden Randbereich aufweist, wobei der Randbereich die zweite Lage und/oder die Zwischenlage umfangsseitig umschließt. In diesem Zusammenhang ist es auch durchaus denkbar, dass der Randbereich der ersten Lage gegenüber der zweiten Lage auf der dem Körper abzuwendenden Seite hervorsteht.

Vorzugsweise sind benachbarte Lagen des Grundkörpers miteinander verklebt.

Um eine besonders gute Anlage des Applikators an der Körperoberfläche zu gewährleisten und ferner den Applikator an dem Körper zu halten, wird es als vorteilhaft angesehen, wenn der Applikator eine bogenförmige, elastisch verformbare Vorspannstruktur aufweist.

Dabei ist es durchaus denkbar, dass die Vorspannstruktur innerhalb des Grundkörpers ausgebildet ist, beispielsweise zwischen unterschiedlichen Lagen des Grundkörpers gehalten ist.

Um eine optimale Anpassung des Applikators an unterschiedliche Körperregionen zu ermöglichen bzw. ein und denselben Grundkörper zu verwenden bzw. den Applikator optimal an die Anatomie des jeweiligen Körpers anzupassen, wird es als vorteilhaft angesehen, wenn die Vorspannstruktur lösbar mit dem Grundkörper verbunden ist. Zur Anpassung des Applikators an die jeweils zu behandelnde Körperregion muss dann lediglich die entsprechende Vorspannstruktur des Applikators ausgetauscht werden und mit dem Grundkörper verbunden werden. So kann beispielsweise eine Anpassung an den Körperumfang erfolgen.

Die Vorspannstruktur lässt sich insbesondere werkzeuglos von dem Grundkörper lösen bzw. mit diesem verbinden.

Vorzugsweise ist die Vorspannstruktur auf der dem Körper abzuwendenden Seite des Applikators angeordnet.

Um ein vollflächiges Andrücken des Grundkörpers an den zu behandelnden Körper zu erreichen, wird es als vorteilhaft angesehen, wenn die Vorspannstruktur plattenförmig ausgebildet ist.

Vorzugsweise weist die Vorspannstruktur eine Aussparung für die Lagerstruktur auf oder zumindest Durchgangsöffnungen für die Anschlüsse auf.

In einer bevorzugten Ausführungsform des Applikators weist der Applikator einen Temperatursensor zur Messung der Temperatur der Körperoberfläche auf. Somit ist es nicht notwendig, zwecks Messung der Temperatur der Körperoberfläche die Behandlung zu unterbrechen und/oder den Applikator von dem Körper zu entfernen, wodurch Behandlungszeit eingespart werden kann. Zudem kann durch die kontinuierliche Temperaturüberwachung der Kühlluftstrom geregelt werden, um eine optimale Oberflächentemperatur zu erreichen. Dies kann durchaus automatisiert erfolgen.

Alternativ oder zusätzlich kann der Applikator auch eine den Applikator von der dem Körper abzuwendenden Seite in Richtung der dem Körper zuzuwendenden Seite durchsetzende Durchgangsöffnung aufweisen. Durch diese Durchgangsöffnung ist es möglich, die Temperatur der Körperoberfläche bei angelegtem Applikator zu messen, beispielsweise mittels eines Infrarotthermometers.

Um ein besonders einfaches Auswechseln der Vorspannstruktur zu ermöglichen, weist der Grundkörper in einer bevorzugten Ausführungsform des Applikators an der dem Körper abzuwendenden Seite einen die Vorspannstruktur umfänglich umschließenden Halteabschnitt, vorzugsweise in Art eines Hinterschnitts, auf. Dadurch, dass der Grundkörper biegsam ist, kann die Vorspannstruktur quasi in den Grundkörper eingeclipst werden.

Vorzugsweise ist der Grundkörper aus einem elastisch verformbaren Material gefertigt, zumindest ist die erste Lage des Grundkörpers vorzugsweise aus einem elastisch verformbaren Material gefertigt. Der Grundkörper oder zumindest die erste Lage des Grundkörpers besteht vorzugsweise aus einem Elastomer. Vorzugsweise besteht der Grundkörper oder zumindest die erste Lage aus Silikon bzw. einem Silikon-Elastomer.

### Figurenbeschreibung

In den Figuren ist die Erfindung anhand eines bevorzugten Ausführungsbeispiels erläutert, ohne hierauf beschränkt zu sein.

Es zeigen:
- Fig. 1: eine perspektivsche Ansicht eines HF-Geräts mit erfindungsgemäßen Applikator in einer perspektivischen Ansicht,
- Fig. 2: der Applikator gemäß Fig. 1 in einer perspektivischen Ansicht,
- Fig. 3: der Applikator gemäß Fig. 1 ohne Halteklammer in einem unverformten Zustand in einer perspektivischen Ansicht,
- Fig. 4: der Applikator in einer Draufsicht gemäß dem Pfeil IV in Fig. 5,
- Fig. 5: der Applikator in einer Schnittansicht gemäß der Linie V-V in Fig. 4,
- Fig. 6: der Applikator gemäß Fig. 3 in einer Explosionsansicht in einer perspektivischen Ansicht,
- Fig. 7: eine erste Lage eines Grundkörpers des Applikators in einer Draufsicht gemäß dem Pfeil VII in Fig. 5,
- Fig. 8: eine Zwischenlage des Grundkörpers des Applikators in einer Ansicht gemäß dem Pfeil VIII in Fig. 5,
- Fig. 9: eine als Düse ausgebildete Luftleiteinrichtung des Applikators in einer Ansicht gemäß dem Pfeil IX in Fig. 10,
- Fig. 10: die Luftleiteinrichtung in einer Schnittansicht gemäß der Linie X-X in Fig. 9,
- Fig. 11: die Halteklammer des Applikators gemäß Fig. 1 in einer perspektivischen Ansicht.

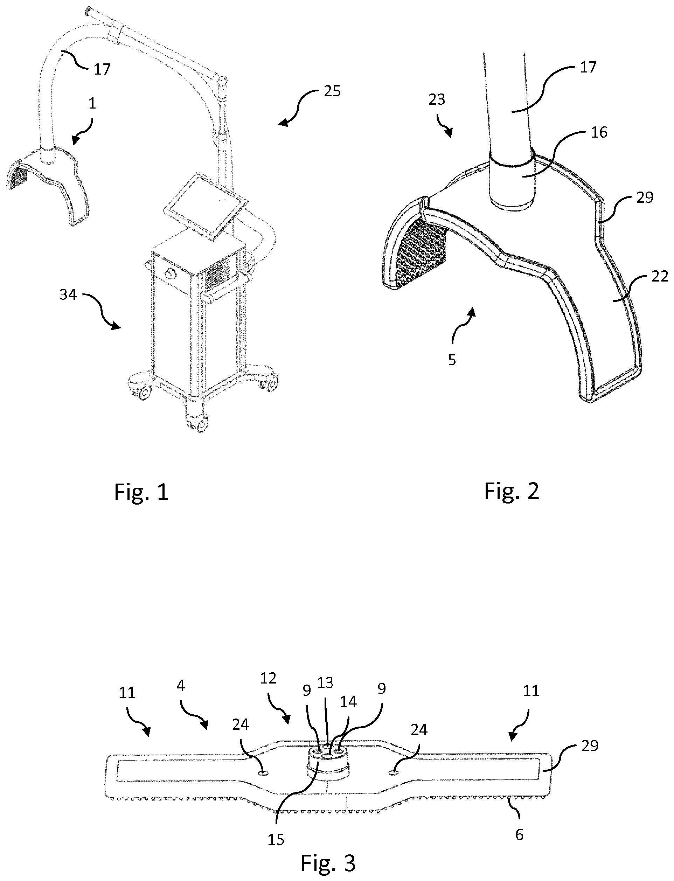

Die Fig. 1 bis 11 zeigen eine Ausführungsform des erfindungsgemäßen Applikators 1 oder Bestandteile des Applikators. Der Applikator 1 dient dem Applizieren eines elektromagnetischen Feldes in Körpergewebe, insbesondere in subkutanes Fettgewebe. Die Fig. 1 zeigt ein Hochfrequenz-Gerät (HF-Gerät) 25, das mit dem Applikator 1 ausgestattet ist.

Der Applikator 1 weist eine biegsame Elektrode 2 und eine biegsame Gegenelektrode 3 auf. Vorliegend handelt es sich bei der Elektrode 2 und die Gegenelektrode 3 um Netzelektroden aus Kupfer. Ferner weist der Applikator 1 einen biegsamen Grundkörper 4 auf, wobei die Elektrode 2 und die Gegenelektrode 3 in dem Grundkörper 4 gelagert sind. Der Applikator 1 weist auf einer dem Körper zuzuwendenden Seite 5 mehrere Abstandshalter 6 auf, wobei die Abstandshalter 6 vorliegend als Noppen ausgebildet und integraler Bestandteil des Grundkörpers 4 sind.

Zwecks Applizieren des elektromagnetischen Feldes in das Körpergewebe wird der Applikator 1 mit seiner dem Körper zuzuwendenden Seite 5 auf den Körper aufgelegt, wobei aufgrund der Biegsamkeit der Elektrode 2, der Gegenelektrode 3 und des Grundkörpers 4 sich der Applikator 1 an die Körperform anpasst, sodass der Applikator 1 sich verformt und im Wesentlichen vollflächig mit der dem Körper zuzuwendenden Seite 5 an dem Körper zur Anlage kommt. Dabei ist es durchaus denkbar, dass bei einer obigen Auflage des Applikators 1 auf dem Körper das Eigengewicht des Applikators 1 bzw. das Eigengewicht des Grundkörpers 4 ausreicht, um die notwendige Anformung an den Körper zu erreichen.

Die Abstandshalter 6 bewirken, dass der Grundkörper 4 an dem Körper anliegt und dementsprechend eine Anformung des Applikators 1 an den Körper erfolgt, allerdings die Elektrode 2 und die Gegenelektrode 3 aufgrund der Abstandshalter 6 in einem definierten Abstand zu dem Körper positioniert sind. Ferner bewirken die Abstandshalter 6, dass zwischen dem Grundkörper 4 und dem Körper eine Luftschicht vorhanden ist und ein Strömen von Luft im Bereich zwischen dem Grundkörper 4 und dem Körper möglich ist. Dabei sind die Abstandshalter 6 derart ausgebildet und angeordnet, dass ein Strömen von Umgebungsluft in den Bereich zwischen der Körperoberfläche und dem Grundkörper 4 und umgekehrt möglich ist. Die Abstandhalter sind vorliegend über die gesamte dem Körper zuzuwendende Seite 5 regelmäßig verteilt ausgebildet.

Der Applikator 1 weist Kanäle 7 auf zum Zuführen und/oder Abführen von Luft auf, wobei der Applikator 1 auf der dem Körper zuzuwendenden Seite mehrere mit den Kanälen 7 fluidverbundene Öffnungen 8 aufweist. Somit ermöglicht es der Applikator 1 aktiv Luft in den Bereich zwischen dem Grundkörper 4 und dem zu behandelnden Körper einzublasen bzw. aus diesem Bereich abzusaugen. Damit kann ein aktives Trocknen der Körperoberfläche und zudem ein Kühlen der Körperoberfläche des zu behandelnden Körpers erfolgen. In diesem Zusammenhang wird es als besonders vorteilhaft angesehen, wenn der aktiv erzeugte Luftstrom derart ist, dass aus den Öffnungen 8 Luft in Richtung der Körperoberfläche strömt und seitlich aus dem Bereich zwischen dem Applikator 1 und der Körperoberfläche in die Umgebung strömt. Besonders vorteilhaft wird es angesehen, wenn es sich bei dem Luftstrom um trockene Luft handelt. Dadurch wird eine besonders gute Trocknungswirkung erreicht. Hinsichtlich einer aktiven Kühlung der Körperoberfläche wird es als vorteilhaft angesehen, wenn die in Richtung der Körperoberfläche strömende Luft eine geringere Temperatur aufweist als die Umgebungsluft.

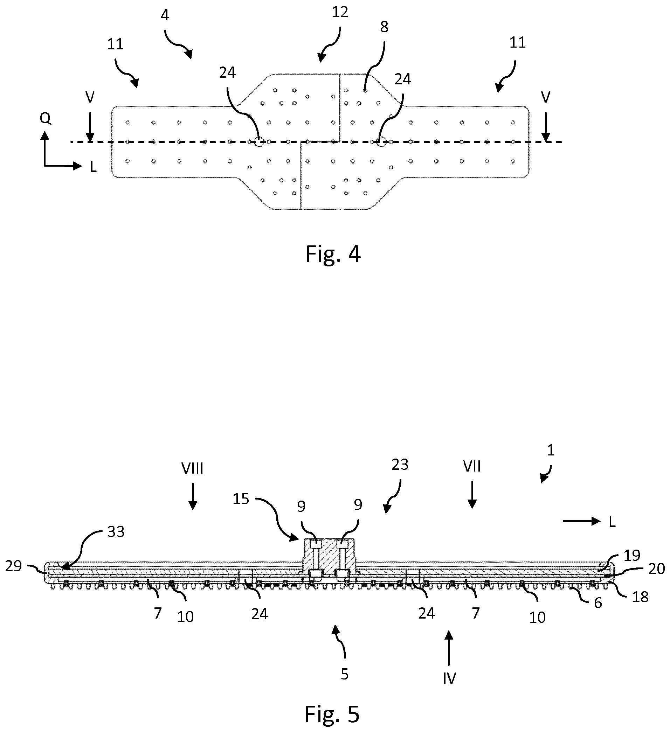

Wie insbesondere der Fig. 4 entnehmbar ist, weist der Grundkörper 4 des Applikators 1 eine Vielzahl von Öffnungen 8 auf. Aus Gründen der Übersichtlichkeit wurde in der Fig. 4 auf eine Darstellung der Abstandshalter 6 verzichtet und es sind lediglich die Öffnung 8 dargestellt. Vorzugsweise weist der Applikator 1 mehr als vierhundert Öffnungen pro Quadratmeter auf, vorliegend achtundsiebzig über die gesamte Fläche verteilte Öffnung 8 auf. Vorliegend weist der Applikator 1 eine Erstreckung in Längsrichtung L von ca. 70 cm und eine Erstreckung in Querrichtung Q von ca. 20 cm auf. Die Erstreckung in Längsrichtung L ist somit größer als die Erstreckung in Querrichtung Q, wobei die Elektrode 2 in der Querrichtung Q beabstandet von der Gegenelektrode 3 angeordnet ist. Vorliegend beträgt der Abstand zwischen den beiden Elektroden 2, 3 ca. 40 mm. Die Elektrode 2 und die Gegenelektrode 3erstrecken sich im Wesentlichen in Längsrichtung L des Applikators 1 und sind bezüglich dieser Längsrichtung L spiegelsymmetrisch zueinander angeordnet.

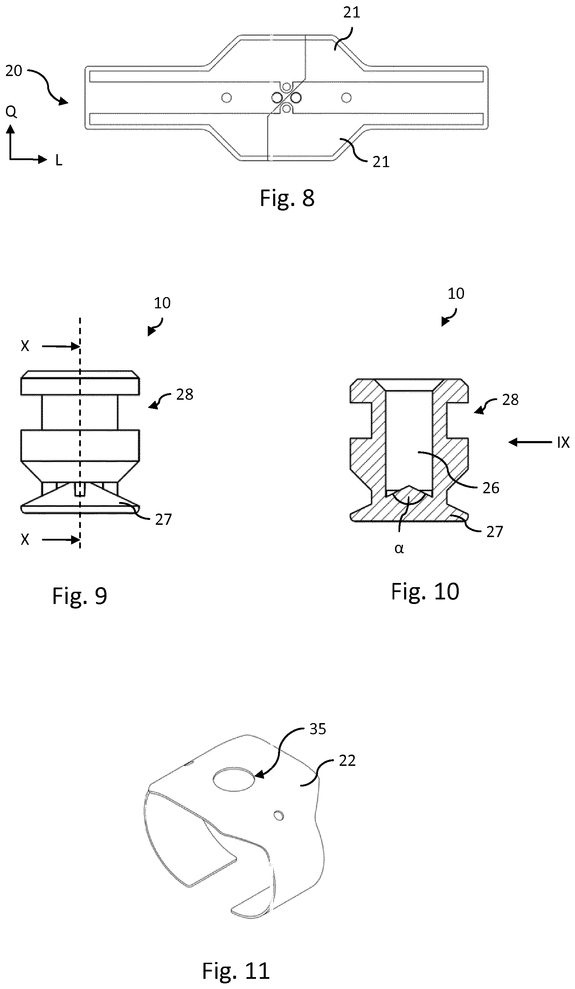

In der jeweiligen Öffnung 8 ist eine Luftleiteinrichtung 10 zum Umlenken und Aufteilen des aus der jeweiligen Öffnung 8 ausströmenden Luftstroms angeordnet. Vorliegend sind die Luftleiteinrichtungen 10 als Düsen ausgebildet, wobei die Fig. 9 und 10 Detailansichten dieser Düse zeigen.

Wie insbesondere den Fig. 9 und 10 zu entnehmen ist, sind die Luftleiteinrichtungen 10 derart ausgebildet, dass der umgelenkte Luftstrom, bezogen auf die Körperoberfläche, eine Tangentialkomponente und eine Normalkomponente aufweist, wobei der Betrag der Tangentialkomponente größer ist als der Betrag der Normalkomponente. Zu diesem Zweck weist die Luftleiteinrichtung 10 eine axiale Durchtrittsöffnung 26 auf, wobei die Luftleiteinrichtung 10 endseitig dieser Durchtrittsöffnung 26 eine konische Umlenkstruktur 27 aufweist, wobei ein Öffnungswinkel α der Umlenkstruktur 27 ca. 130° beträgt.

Die jeweilige Luftleiteinrichtung 10 ist als separates Teil ausgebildet und lösbar im Bereich der jeweiligen Öffnung 8 gelagert. Zu diesem Zweck weist die Luftleiteinrichtung 10 eine Lagerstruktur 28 in Art einer umlaufenden Nut auf. Zwecks Lagerung der Luftleiteinrichtung 10 im Bereich der jeweiligen Öffnung 8 wird die Luftleiteinrichtung 10 derart in den Grundkörper 4 eingebracht, dass ein Teilbereich des Grundkörpers 4 in die Nut eingreift, sodass die jeweilige Luftleiteinrichtung 10 hinterschnittartig in dem Grundkörper 4 gehalten ist.

Wie insbesondere der Fig. 3 zu entnehmen ist, weist der Applikator 1 einen zwischen zwei Endabschnitten 11 ausgebildeten zentralen Abschnitt 12 auf, wobei der Applikator 1 in diesem zentralen Abschnitt 12 eine größere Erstreckung in Querrichtung Q aufweist als im Bereich der beiden Endabschnitte 11. Ferner sind die Elektrode 2 und die Gegenelektrode 3 derart ausgebildet, dass bei Verwendung des Applikators 1 im Bereich des zentralen Abschnitts 12 des Applikators 1 eine stärkere Erwärmung des Körpergewebes erreicht werden kann als im Bereich der beiden Endabschnitte 11. Zu diesem Zweck sind die Elektrode 2 und die Gegenelektrode 3 im Bereich des zentralen Abschnitts 12 flächenmäßig größer ausgebildet als im Bereich der beiden Endabschnitte 11. Der Applikator 1 dient vorliegend insbesondere zur Verwendung im Bereich eines Torsos, insbesondere im Bereich des Bauchs einer Person, sodass bei Bauchanlage sowohl der Rippenbogen der zu behandelnden Person als auch der Hüftknochen besser ausgespart werden, insofern in diesen Bereichen eine geringere Erwärmung aufgrund des Applizierens des elektromagnetischen Feldes erfolgt als dies im Bereich des Bauchs der Fall ist. Ein wesentlicher Vorteil der Ausrichtung der Elektrode und Gegenelektrode senkrecht zur Körperachse (eine Elektrode oben, eine unten) ist, dass dadurch die Randbereiche wenig erwärmt werden. Speziell bei der Bauchanlage ist so sichergestellt, dass die eher empfindlichen Knochen mit Knochenmark (Rippenbogen, Hüftknochen) nicht unnötig erwärmt werden.

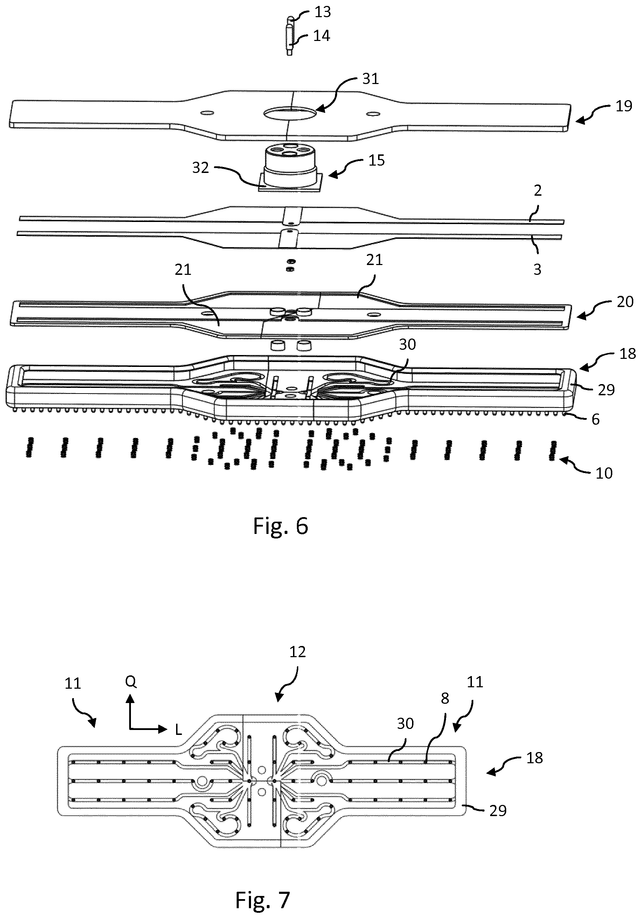

Wie aus der Explosionsdarstellung des Applikators 1 in der Fig. 6 sowie der Fig. 5 hervorgeht, ist der Grundkörper 4 aus mehreren Lagen 18, 19, 20 gebildet, vorliegend aus drei Lagen 18, 19, 20 gebildet, wobei eine erste Lage 18 des Grundkörpers 4 auf einer ersten Seite 5, vorliegend der dem Körper zuzuwendenden Seite 5, mehrere Vorsprünge aufweist. Diese Vorsprünge bilden vorliegend die Abstandshalter 6. Ferner weist die erste Lage 18 auf einer der ersten Seite 5 abgewandten Seite 23, vorliegend der dem Körper abzuwendenden Seite 23, mehrere Rücksprünge 30 auf, wobei die Rücksprünge 30 Teilbereiche der Kanäle 7 bilden. Auf der zweiten Seite 23 der ersten Lage 18 liegt eine Zwischenlage 20 auf, wobei die Zwischenlage 20 die offenen Rücksprünge 30 der ersten Lage 18 abdeckt, wodurch die Kanäle 7 gebildet sind. Die Zwischenlage 20 weist zwei Aussparungen 21 auf, wobei die Elektrode 2 und die Gegenelektrode 3 jeweils in einer der zwei Aussparungen 21 angeordnet sind. Ferner weist der Grundkörper 4 eine zweite Lage 19 auf, wobei die Zwischenlage 20 zwischen der ersten Lage 18 und der zweite Lage 19 angeordnet ist. Somit deckt die zweite Lage 19 die Elektrode 2 und die Gegenelektrode 3 auf der dem Körper abzuwendenden Seite 23 des Applikators 1 ab. Die erste Lage 18, die Zwischenlage 20 und die zweite Lage 19 sind miteinander verklebt.

Der Applikator 1 weist einen ersten elektrischen Anschluss 13 für die Elektrode 2, einen zweiten elektrischen Anschluss 14 für die Gegenelektrode 3 und zwei Anschlüsse 9 zum Zuführen und/oder Abführen von Luft in die Kanäle 7 und/oder aus den Kanälen 7 auf. Sämtliche Anschlüsse 9, 13, 14 sind vorliegend in einer gemeinsamen Lagerstruktur 15 gelagert. Diese Lagerstruktur 15 ist als Buchse ausgebildet, wobei ein Lagerabschnitt 32 dieser Buchse 15 zwischen der zweiten Lage 19 und der Zwischenlage 20 angeordnet ist. Ein Teilbereich der Lagerstruktur 15 durchsetzt eine in der zweiten Lage 19 ausgebildete Durchgangsöffnung 31. Die Lagerstruktur 15 ist vorliegend in dem zentralen Abschnitt 12 des Applikators 1 angeordnet.

Aufgrund der gemeinsamen Lagerung der Anschlüsse 9, 13, 14 in der Lagerstruktur 15 ist die Handhabung des Applikators 1 besonders einfach, da zum Betrieb des Applikators 1 bzw. zum Verbinden des Applikators 1 mit einer Hochfrequenz-Quelle und einer Luftzufuhr- bzw. -abführeinrichtung lediglich ein einziger Stecker 16 mit der Lagerstruktur 15 verbunden werden muss, wie dies in der Fig. 1 dargestellt ist. Zu diesem Zweck ist die Lagerstruktur 15 als Buchse ausgebildet, in welche der Stecker 16 einsteckbar ist. Der Stecker 16 ist mit einer ersten elektrischen Leitung für die Elektrode 2, einer zweiten elektrischen Leitung für die Gegenelektrode 3 und zumindest einer Leitung für die zwei Anschlüsse 9 verbunden. Sämtliche Leitungen sind in einer schlauchartigen, biegsamen Führungsstruktur 17 gelagert, wobei die Führungsstruktur 17 vorliegend einen ersten Kanal für die erste elektrische Leitung und einen zweiten Kanal für die zweite elektrische Leitung aufweist, um die elektrischen Leitungen definiert und beabstandet voneinander zu führen.

Vorliegend weist der Applikator 1 eine bogenförmige, elastisch verformbare Vorspannstruktur 22 auf, wobei diese Vorspannstruktur 22 lösbar mit dem Grundkörper 4 verbunden ist und eine Aussparung 35 für die Lagerstruktur 15 aufweist. Zwecks lösbarem Verbindens der Vorspannstruktur 22 mit dem Grundkörper 4 weist der Grundkörper 4, nämlich die erste Lage 18, einen umlaufenden Rand 29 auf, wobei ein Teilbereich des Rands 29 auf einer dem Körper abgewandten Seite 23 umfänglich hervorsteht und einen als Hinterschnitt ausgebildeten Halteabschnitt 33 aufweist, wobei die Vorspannstruktur 22 in diesen Hinterschnitt einführbar ist, um die Vorspannstruktur 22 mit dem Grundkörper 4 zu verbinden. Durch die gebogene Form der gegenüber dem Grundkörper 4 und den Elektroden 2, 3 steiferen Vorspannstruktur 22 ist der gesamte Applikator 1 in Art eines Bogens vorgeformt bzw. vorgespannt. Die dem Applikator 1 durch die Vorspannstruktur 22 aufgeprägte Form ist vorliegend derart, dass der Applikator 1 etwa der Form des Torsos einer Person angepasst ist. Zwecks Verbindens des Applikators 1 mit der zu behandelnden Person wird der Applikator 1 entgegen der Rückstellkraft der Vorspannstruktur 22 etwas aufgebogen und um den Torso der Person herumgelegt. Aufgrund der elastischen Verformung legt sich der Applikator 1, nämlich der biegsame Grundkörper 4 mit den darin gelagerten Elektroden 2, 3 vollflächig an den Körper der Person an und ist in seiner Position gehalten. Die zu behandelnde Person kann somit auch Bewegungen ausführen, ohne den Elektroden-Haut-Abstand wesentlich zu verändern.

Vorliegend bestehen die erste Lage 18, die Zwischenlage 20 und die zweite Lage 19 des Grundkörpers 4 aus einem Elastomer, nämlich einem Silikon-Elastomer. Die Lagerstruktur besteht vorliegend aus Polypropylen. Die Vorspannstruktur 22 besteht vorzugsweise aus einem Kunststoff, insbesondere aus einem elastischen Kunststoff. Als besonders vorteilhaft hat sich die Verwendung eines ABS-Kunststoffs für die Vorspannstruktur 22 erwiesen.

Um in einfacher Art und Weise eine Temperaturmessung der Körperoberfläche, beispielsweise mittels eines Infrarotthermometers, zu ermöglichen, weist der Applikator 1 eine den Applikator 1 von der dem Körper abzuwendenden Seite 23 in Richtung der den Körper zuzuwendenden Seite 5 durchsetzende Durchgangsöffnung 24 auf. Vorzugsweise weist das HF-Gerät 25 sowohl die HF-Quelle als auch eine Luftfördereinrichtung, vorzugsweise eine Luftzufuhreinrichtung, bspw. einen Verdichter bzw. Kompressor auf. Vorzugsweise weist das HF-Gerät 25 eine Kühleinrichtung zum Kühlen der dem Applikator 1 zugeführten Luft auf. Es ist durchaus denkbar, dass die HF-Quelle und die Luftfördereinrichtung sowie die Kühleinrichtung in einem gemeinsamen Gehäuse 34 gelagert sind. Ferner kann dieses Gehäuse 34 auch der Aufnahme einer Steuereinrichtung des HF-Geräts 25 dienen. Als besonders vorteilhaft wird es angesehen, wenn das HF-Gerät 25 als mobiles Gerät ausgebildet ist.

### Bezugszeichenliste

- 1: Applikator
- 2: Elektrode
- 3: Gegenelektrode
- 4: Grundkörper
- 5: Seite
- 6: Abstandshalter
- 7: Kanal
- 8: Öffnung
- 9: Anschluss
- 10: Luftleiteinrichtung
- 11: Endabschnitt
- 12: zentraler Abschnitt
- 13: erster elektrischer Anschluss
- 14: zweiter elektrischer Anschluss
- 15: Lagerstruktur
- 16: Stecker
- 17: Führungsstruktur
- 18: erste Lage
- 19: zweite Lage
- 20: Zwischenlage
- 21: Aussparung
- 22: Vorspannstruktur
- 23: Seite
- 24: Durchgangsöffnung
- 25: HF-Gerät
- 26: Durchtrittsöffnung
- 27: Umlenkstruktur
- 28: Lagerstruktur
- 29: Rand
- 30: Rücksprung
- 31: Durchgangsöffnung
- 32: Lagerabschnitt
- 33: Halteabschnitt
- 34: Gehäuse
- 35: Aussparung

- L: Längsrichtung
- Q: Querrichtung
- α: Winkel

## Patentansprüche

1. Applikator (1) zum Applizieren eines elektromagnetischen Feldes in Körpergewebe, insbesondere in subkutanes Fettgewebe, wobei der Applikator (1) zumindest eine Elektrode (2) und zumindest eine Gegenelektrode (3) aufweist, wobei der Applikator (1) einen biegsamen Grundkörper (4) aufweist, wobei die zumindest eine Elektrode (2) und die zumindest eine Gegenelektrode (3) in dem Grundkörper (4) gelagert sind, wobei der Applikator (1) auf einer dem Körper zuzuwendenden Seite (5) mehrere Abstandshalter (6) aufweist, **dadurch gekennzeichnet, dass** der Applikator (1) Kanäle (7) zum Zuführen und/oder Abführen von Luft aufweist, wobei der Applikator (1) auf der dem Körper zuzuwendenden Seite (5) mehrere mit den Kanälen (7) fluidverbundene Öffnungen (8) aufweist.

2. Applikator nach Anspruch 1, wobei der Applikator (1) zumindest einen Anschluss (9) aufweist, wobei der Anschluss (9) mit den Kanälen (7) fluidverbunden ist, wobei der Anschluss (9) mit einer Einrichtung zum Zuführen und/oder Abführen von Luft verbindbar ist.

3. Applikator nach Anspruch 1 oder 2, wobei in der jeweiligen Öffnung (8) eine Luftleiteinrichtung (10) zum Umlenken und/oder Aufteilen des Luftstroms angeordnet ist.

4. Applikator nach Anspruch 3, wobei die Luftleiteinrichtung (10) als separates Teil ausgebildet und vorzugsweise lösbar in der Öffnung (8) gelagert ist.

5. Applikator nach einem der Ansprüche 1 bis 4, wobei die Abstandshalter (6) derart ausgebildet sind, dass bei Anlage des Applikators (1) an dem Körper ein Strömen von Luft von außerhalb in den Bereich zwischen Applikator (1) und Körperoberfläche und/oder ein Strömen von Luft zwischen Applikator (1) und Körperoberfläche nach außerhalb möglich ist.

6. Applikator nach einem der Ansprüche 1 bis 5, wobei der Applikator (1) eine Erstreckung in Längsrichtung (L) und eine Erstreckung in Querrichtung (Q) aufweist, wobei die Erstreckung in Längsrichtung (L) größer ist als die Erstreckung in Querrichtung (Q), wobei die Elektrode (2) in der Querrichtung (Q) beabstandet von der Gegenelektrode (3) angeordnet ist.

7. Applikator nach einem der Ansprüche 1 bis 6, wobei die Elektrode (2) als biegsame Elektrode (2) ausgebildet ist und/oder die Gegenelektrode (3) als biegsame Gegenelektrode (3) ausgebildet ist, vorzugsweise die Elektrode (2) und die Gegenelektrode (3) als biegsame Metallstreifen und/oder Netzelektroden und/oder Folienelektroden ausgebildet sind.

8. Applikator nach einem der Ansprüche 1 bis 7, wobei der Applikator (1) einen zwischen zwei Endabschnitten (11) ausgebildeten zentralen Abschnitt (12) aufweist, wobei die Elektrode (2) und die Gegenelektrode (3) derart ausgebildet sind, dass bei Verwendung des Applikators (1) im Bereich des zentralen Abschnitts (12) des Applikators (1) eine stärkere Erwärmung des Körpergewebes erreichbar ist als im Bereich der beiden Endabschnitte (11).

9. Applikator nach einem der Ansprüche 1 bis 8, wobei der Applikator (1) einen ersten elektrischen Anschluss (13) für die Elektrode (2), einen zweiten elektrischen Anschluss (14) für die Gegenelektrode (3) und zumindest einen Anschluss (9) zum Zuführen und/oder Abführen von Luft in die Kanäle (7) und/oder aus den Kanälen (7) aufweist, wobei sämtliche Anschlüsse (9, 13, 14) in einer gemeinsamen Lagerstruktur (15) gelagert sind.

10. Applikator nach Anspruch 9, wobei die Lagerstruktur (15) als Buchse ausgebildet ist, wobei ein Stecker (16) lösbar mit der Buchse verbindbar ist, wobei der Stecker (16) mit einer ersten elektrischen Leitung für die Elektrode (2), einer zweiten elektrischen Leitung für die Gegenelektrode (3) und einer Leitung für den zumindest einen Anschluss (9) zum Zuführen und/oder Abführen von Luft verbunden ist, wobei sämtliche Leitungen in einer schlauchartigen, biegsamen Führungsstruktur (17) gelagert sind, wobei die Führungsstruktur (17) einen ersten Kanal für die erste elektrische Leitung und einen zweiten Kanal für die zweite elektrische Leitung aufweist, oder wobei die Lagerstruktur (15) als Stecker ausgebildet ist, wobei eine Buchse lösbar mit dem Stecker verbindbar ist, wobei die Buchse mit einer ersten elektrischen Leitung für die Elektrode (2), einer zweiten elektrischen Leitung für die Gegenelektrode (3) und einer Leitung für den zumindest einen Anschluss (9) zum Zuführen und/oder Abführen von Luft verbunden ist, wobei sämtliche Leitungen in einer schlauchartigen, biegsamen Führungsstruktur (17) gelagert sind, wobei die Führungsstruktur (17) einen ersten Kanal für die erste elektrische Leitung und einen zweiten Kanal für die zweite elektrische Leitung aufweist.

11. Applikator nach einem der Ansprüche 1 bis 10, wobei der Grundkörper (4) aus mehreren Lagen (18, 19, 20) gebildet ist, wobei eine erste Lage (18) des Grundkörpers (4) auf einer ersten Seite (5) mehrere Vorsprünge aufweist, wobei die Vorsprünge die Abstandshalter (6) bilden, und wobei die erste Lage (18) auf einer der ersten Seite (5) abgewandten Seite (23) mehrere Rücksprünge (30) aufweist, wobei die Rücksprünge (30) Teilbereiche der Kanäle (7) bilden.

12. Applikator nach Anspruch 11, wobei der Grundkörper (4) eine zweite Lage (19) aufweist, wobei die Elektrode (2) und die Gegenelektrode (3) zwischen der ersten Lage (18) und der zweiten Lage (19) angeordnet sind.

13. Applikator nach einem der Ansprüche 1 bis 12, wobei der Applikator (1) eine bogenförmige, elastisch verformbare Vorspannstruktur (22) aufweist.

14. Applikator nach Anspruch 13, wobei die Vorspannstruktur (22) lösbar mit dem Grundkörper (4) verbunden ist.

15. Applikator nach einem der Ansprüche 13 oder 14, wobei der Grundkörper (4) auf einer dem Körper abzuwendenden Seite (23) einen die Vorspannstruktur (22) umfänglich umschließenden Halteabschnitt (33) aufweist.

## Claims

1. Applicator (1) for applying an electromagnetic field in body tissue, in particular in subcutaneous adipose tissue, wherein the applicator (1) has at least one electrode (2) and at least one counter electrode (3), wherein the applicator (1) has a flexible main body (4), wherein the at least one electrode (2) and the at least one counter electrode (3) are mounted in the main body (4), wherein the applicator (1) has a plurality of spacers (6) on a side (5) to be turned towards the body, **characterized in that** the applicator (1) has channels (7) for supplying and/or discharging air, wherein the applicator (1) has a plurality of openings (8), which are fluidically connected to the channels (7), on the side (5) to be turned towards the body.

2. Applicator according to Claim 1, wherein the applicator (1) has at least one connection (9), wherein the connection (9) is fluidically connected to the channels (7), wherein the connection (9) can be connected to a device for supplying and/or discharging air.

3. Applicator according to Claim 1 or 2, wherein an air guide device (10) for deflecting and/or splitting the air flow is arranged in the respective opening (8).

4. Applicator according to Claim 3, wherein the air guiding device (10) is in the form of a separate part and is preferably detachably mounted in the opening (8).

5. Applicator according to any of Claims 1 to 4, wherein the spacers (6) are formed in such a way that, when the applicator (1) makes contact with the body, it is possible for air to flow from the outside into the region between the applicator (1) and the body surface and/or for air between the applicator (1) and the body surface to flow to the outside.

6. Applicator according to any of Claims 1 to 5, wherein the applicator (1) has an extent in the longitudinal direction (L) and an extent in the transverse direction (Q), wherein the extent in the longitudinal direction (L) is greater than the extent in the transverse direction (Q), wherein the electrode (2) is arranged at a distance from the counter electrode (3) in the transverse direction (Q).

7. Applicator according to any of Claims 1 to 6, wherein the electrode (2) is in the form of a flexible electrode (2) and/or the counter electrode (3) is in the form of a flexible counter electrode (3), the electrode (2) and the counter electrode (3) preferably being in the form of flexible metal strips and/or mesh electrodes and/or foil electrodes

8. Applicator according to any of Claims 1 to 7, wherein the applicator (1) has a central portion (12) formed between two end portions (11), wherein the electrode (2) and the counter electrode (3) are formed in such a way that, when the applicator (1) is in use, the body tissue can be heated more intensely in the region of the central portion (12) of the applicator (1) than in the region of the two end portions (11).

9. Applicator according to any of Claims 1 to 8, wherein the applicator (1) has a first electrical connection (13) for the electrode (2), a second electrical connection (14) for the counter electrode (3) and at least one connection (9) for supplying and/or discharging air into the channels (7) and/or from the channels (7), wherein all the connections (9, 13, 14) are mounted in a common mounting structure (15).

10. Applicator according to Claim 9, wherein the mounting structure (15) is in the form of a socket, wherein a plug (16) can be releasably connected to the socket, wherein the plug (16) is connected to a first electrical line for the electrode (2), a second electrical line for the counter electrode (3) and a line for the at least one connection (9) for supplying and/or discharging air, wherein all the lines are mounted in a tubular, flexible guide structure (17), wherein the guide structure (17) has a first channel for the first electrical line and a second channel for the second electrical line, or wherein the mounting structure (15) is in the form of a plug, wherein a socket can be releasably connected to the plug, wherein the socket is connected to a first electrical line for the electrode (2), a second electrical line for the counter electrode (3) and a line for the at least one connection (9) for supplying and/or discharging air, wherein all the lines are mounted in a tubular, flexible guide structure (17), wherein the guide structure (17) has a first channel for the first electrical line and a second channel for the second electrical line.

11. Applicator according to any of Claims 1 to 10, wherein the main body (4) is formed from several layers (18, 19, 20), wherein a first layer (18) of the main body (4) has several projections on a first side (5), wherein the projections form the spacers (6), and wherein the first layer (18) has several recesses (30) on a side (23) facing away from the first side (5), wherein the recesses (30) form subregions of the channels (7).

12. Applicator according to Claim 11, wherein the main body (4) has a second layer (19), wherein the electrode (2) and the counter electrode (3) are arranged between the first layer (18) and the second layer (19).

13. Applicator according to any of Claims 1 to 12, wherein the applicator (1) has an arcuate, elastically deformable preloading structure (22).

14. Applicator according to Claim 13, wherein the preloading structure (22) is releasably connected to the main body (4).

15. Applicator according to either of Claims 13 and 14, wherein the main body (4) has a holding portion (33) circumferentially surrounding the preloading structure (22) on a side (23) to be turned away from the body.

## Revendications

1. Applicateur (1) pour appliquer un champ électromagnétique dans un tissu corporel, en particulier dans un tissu adipeux sous-cutané, l'applicateur (1) comportant au moins une électrode (2) et au moins une contre-électrode (3), l'applicateur (1) comportant un corps principal (4) flexible, l'au moins une électrode (2) et l'au moins une contre-électrode (3) étant montées dans le corps principal (4), l'applicateur (1) comportant plusieurs entretoises (6) sur un côté (5) devant être tourné vers le corps, **caractérisé en ce que** l'applicateur (1) comporte des canaux (7) pour amener et/ou évacuer de l'air, l'applicateur (1) comportant, sur le côté (5) devant être tourné vers le corps, plusieurs ouvertures (8) en communication fluidique avec les canaux (7).

2. Applicateur selon la revendication 1, l'applicateur (1) comportant au moins un raccord (9), le raccord (9) étant en communication fluidique avec les canaux (7), le raccord (9) pouvant être relié à un dispositif destiné à amener et/ou évacuer de l'air.

3. Applicateur selon la revendication 1 ou 2, **caractérisé en ce qu'**un dispositif de guidage d'air (10) destiné à dévier et/ou diviser le flux d'air est disposé dans l'ouverture (8) respective.

4. Applicateur selon la revendication 3, le dispositif de guidage d'air (10) étant formé comme une pièce séparée et étant monté de préférence de manière amovible dans l'ouverture (8).

5. Applicateur selon l'une des revendications 1 à 4, les entretoises (6) étant formées de telle manière que, lorsque l'applicateur (1) est en contact avec le corps, un écoulement d'air de l'extérieur dans la zone entre l'applicateur (1) et la surface du corps et/ou un écoulement d'air entre l'applicateur (1) et la surface du corps vers l'extérieur sont possibles.

6. Applicateur selon l'une des revendications 1 à 5, l'applicateur (1) présentant une extension dans la direction longitudinale (L) et une extension dans la direction transversale (Q), l'extension dans la direction longitudinale (L) étant plus grande que l'extension dans la direction transversale (Q), l'électrode (2) étant disposée à distance de la contre-électrode (3) dans la direction transversale (Q).

7. Applicateur selon l'une des revendications 1 à 6, l'électrode (2) étant formée comme une électrode flexible (2) et/ou la contre-électrode (3) étant formée comme une contre-électrode flexible (3), de préférence l'électrode (2) et la contre-électrode (3) étant formées comme des bandes métalliques flexibles et/ou des grilles à mailles et/ou des électrodes à film.

8. Applicateur selon l'une des revendications 1 à 7, l'applicateur (1) comportant une section centrale (12) formée entre deux sections d'extrémité (11), l'électrode (2) et la contre-électrode (3) étant formées de telle manière que, lors de l'utilisation de l'applicateur (1), un réchauffement plus important des tissus corporels dans la zone de la section centrale (12) de l'applicateur (1) que dans la zone des deux sections d'extrémité (11) peut être atteint.

9. Applicateur selon l'une des revendications 1 à 8, l'applicateur (1) comportant un premier raccord électrique (13) pour l'électrode (2), un deuxième raccord électrique (14) pour la contre-électrode (3) et au moins un raccord (9) pour l'amenée et/ou l'évacuation d'air dans les canaux (7) et/ou hors des canaux (7), tous les raccords (9, 13, 14) étant montés dans une structure de support commune (15).

10. Applicateur selon la revendication 9, la structure de support (15) étant formée comme une prise femelle, une prise mâle (16) pouvant être reliée de manière amovible à la prise femelle, la prise mâle (16) étant reliée à une première ligne électrique pour l'électrode (2), à une deuxième ligne électrique pour la contre-électrode (3) et à une ligne pour l'au moins un raccord (9) destiné à amener et/ou évacuer de l'air, toutes les lignes étant montées dans une structure de guidage (17) flexible en forme de tuyau, la structure de guidage (17) comportant un premier canal pour la première ligne électrique et un deuxième canal pour la deuxième ligne électrique, ou la structure de support (15) étant formée comme une fiche mâle, une fiche femelle pouvant être reliée de manière amovible à la fiche mâle, la fiche femelle étant reliée à une première ligne électrique pour l'électrode (2), à une deuxième ligne électrique pour la contre-électrode (3) et à une ligne pour l'au moins un raccord (9) destiné à amener et/ou évacuer de l'air, toutes les lignes étant montées dans une structure de guidage (17) flexible en forme de tuyau, la structure de guidage (17) comportant un premier canal pour la première ligne électrique et un deuxième canal pour la deuxième ligne électrique.

11. Applicateur selon l'une des revendications 1 à 10, le corps principal (4) étant formé à partir de plusieurs couches (18, 19, 20), une première couche (18) du corps principal (4) comportant plusieurs saillies sur un premier côté (5), les saillies formant les entretoises (6), et la première couche (18) comportant plusieurs retraits (30) sur un côté (23) opposé au premier côté (5), les retraits (30) formant des zones partielles des canaux (7).

12. Applicateur selon la revendication 11, le corps principal (4) comportant une deuxième couche (19), l'électrode (2) et la contre-électrode (3) étant disposées entre la première couche (18) et la deuxième couche (19).

13. Applicateur selon l'une des revendications 1 à 12, l'applicateur (1) comportant une structure de précontrainte (22) en forme d'arc, élastiquement déformable.

14. Applicateur selon la revendication 13, la structure de précontrainte (22) étant reliée de manière amovible au corps principal (4).

15. Applicateur selon l'une des revendications 13 ou 14, le corps principal (4) comportant, sur un côté (23) destiné à être tourné à l'opposé du corps, une section de maintien (33) entourant en périphérie la structure de précontrainte (22).
